(19) Europäisches Patentamt
European Patent Office
Office européen des brevets

(11) Publication number : **0 634 174 A1**

(12) **EUROPEAN PATENT APPLICATION**

(21) Application number : **94305093.0**

(51) Int. Cl.$^6$ : **A61K 31/70**, A61K 33/26

(22) Date of filing : **12.07.94**

(30) Priority : **13.07.93 JP 173122/93**

(43) Date of publication of application :
**18.01.95 Bulletin 95/03**

(84) Designated Contracting States :
**AT BE CH DE DK ES FR GB GR IE IT LI LU NL PT SE**

(71) Applicant : **Takeda Chemical Industries, Ltd.**
**1-1 Doshomachi 4-chome,**
**Chuo-ku**
**Osaka-shi, Osaka 541 (JP)**

(72) Inventor : **Ishiguro, Toshihiro**
**12-9, Kofudai 4-chome,**
**Toyono-cho**
**Toyono-gun, Osaka 563-01 (JP)**
Inventor : **Narukawa, Noriaki**
**205-8, Aza-osada,**
**Fukuchiyama**
**Kyoto 620-11 (JP)**
Inventor : **Oka, Masahide**
**4-141, Seiwadainishi 4-chome,**
**Kawanishi**
**Hyogo 666-01 (JP)**
Inventor : **Yamamoto, Hiromi**
**174-8, Aza-daitou,**
**Miwa-cho**
**Amata-gun, Kyoto 620-13 (JP)**

(74) Representative : **Lewin, John Harvey**
**Elkington and Fife**
**Prospect House**
**8 Pembroke Road**
**Sevenoaks, Kent TN13 1XR (GB)**

(54) **Antianemic composition for veterinary use.**

(57) An antianemic composition for veterinary use which comprises a complex of a dextran carboxylic acid obtained by a microbial process and an iron salt, and a veterinary acceptable carrier is disclosed. The composition can contain a high concentration of iron and have a low viscosity and particularly useful for preventing or treating anemia of piglets caused by iron deficiency.

EP 0 634 174 A1

## FIELD OF THE INVENTION

The present invention relates to an antianemic composition for veterinary use (hereinafter referred to as an antianemic veterinary composition). More specifically, it relates to a veterinary composition for supplying animals with iron to prevent or treat anemia of the animals. In particular, the composition of the present invention is useful for preventing or treating anemia of piglets caused by iron deficiency.

## BACKGROUND OF THE INVENTION

It is well known that piglets are liable to anemia caused by iron deficiency within a short period of time after their birth because of their little iron storage due to their small birth weights, and insufficient iron supply due to their quick growth and low iron content of their sow's milk. In particular, recently, as rearing of pigs in a closed pen system has become popular, iron ingestion from the earth has become difficult and the frequency of occurrence of anemia caused by iron deficiency in piglets, in particular, suckling pigs is increased.

Hitherto, ferrous fumarate, dextran iron and the like have been administered to exert antianemic activity on these animals.

Dextran is a general name of high molecular weight glucans mainly composed of $\alpha$ 1→6 bonds and produced from sucrose by a lactobacillus, Leuconostoc mesenteroides. Dextran-iron which is produced from dextran and iron hydroxide has been used as a prophylactically or therapeutically effective product for supplying animals, in particular, piglets with iron by parenteral administration such as intramuscular injection to improve anemic disease conditions caused by iron deficiency.

Especially, a dextran-iron preparation comprising a complex of dextran and ferric hydroxide as well as a preparation comprising a complex of ferric hydroxide and dextran heptonic acid which is obtained from dextran by a chemical reaction for increasing its carbon atom have been utilized as prophylactic or therapeutic agents for anemia of pigs caused by iron deficiency.

On the other hand, although various chemical modifications of dextran have been attempted, it is very difficult to obtain products regio-selectively in high yields by generally adopted chemical reactions and many by-products are formed. That is, since dextran and many other polysaccharides are composed of various high molecular weight congeners having different molecular weights, in chemical modification, various side reactions take place, which results in the formation of complicated products and, in many cases, difficulties arise in identification of the reaction products (Biotransformations in Preparative Organic Chemistry, H.G. Davis et al., Academic Press).

More specifically, although chemical oxidization of dextran with an oxidizing agent such as sodium hypochlorite, sodium bromite, chlorine, bromine, iodine or the like has been attempted (for example, JP-A 61-233001), the structures of products are not necessarily identified or fully supported.

In known antianemic preparations, it is difficult to stably formulate iron at a high concentration with maintaining a low viscosity and there is a problem that a colored spot is formed at an intramuscular injected site. These difficulties and problem are considered to be caused by relatively low purity of modified dextrans per se which are derived from dextran by chemical synthetic means, in particular, the presence of by-products due to side reactions accompanied by chemical reactions.

## OBJECTS OF THE INVENTION

The main object of the present invention is to provide an antianemic veterinary composition which has more improved effects and less toxicity and side-effects in comparison with conventional agents for preventing and treating anemia of animals.

This object and other objects and advantages of the present invention will become apparent to those skilled in the art from the following description.

## SUMMARY OF THE INVENTION

Under these circumstances, the present inventors have intensively investigated and studied various compounds having antianemic activities. As a result, it has been found that a complex of an iron slat with a dextran carboxylic acid obtained by a microbial process in high purity improves anemia of animals such as pigs.

That is, according to the present invention, there is provided an antianemic veterinary composition which comprises a complex of a dextran carboxylic acid obtained by a microbial process with an iron salt, and, if necessary, a veterinary acceptable carrier. In particular, the dextran carboxylic acid to be used in the antianemic veterinary composition of the present invention is obtained by converting a dextran having hydroxymethyl

group and/or hemiacetal hydroxyl group to the corresponding carboxylic acid or its salt with a microorganism, which belongs to the genus Pseudogluconobacter and is capable of oxidizing the hydroxymethyl group and/or the OH-containing hemiacetal moiety of the dextran to carboxyl group, or a processed material thereof, and recovering the resulting corresponding carboxylic acid or its salt.

## DETAILED DISCLOSURE OF THE INVENTION

Conventional microbial oxidization of saccharides and other compounds having hydroxymethyl group and/or OH-containing hemiacetal moiety to the corresponding carboxylic acids is restrictedly applied to monosaccharide because of the substrate specificity of a microorganism to be used. In addition, as described above, many side reactions are involved in chemical oxidization of hydroxymethyl group and OH-containing hemiacetal moiety, which unavoidably requires complicated purification steps. Then, it has been desired to develop a process for the production of an oxidized dextran efficiently with high selectivity, and to produce dextran carboxylic acids having high purity.

According to the present invention, a dextran carboxylic acid having high purity can be obtained in high yield and selectivity by oxidizing hydroxymethyl group and/or OH-containing hemiacetal moiety of a dextran with a microorganism.

The microorganism to be used for the production of a dextran carboxylic acid by a microbial process in the present invention may be any microorganism belonging to the genus Pseudogluconobacter in so far as it is capable of oxidizing hydroxymethyl group and/or OH-containing hemiacetal moiety of a saccharide, including mutants thereof obtained by normal mutagenesis procedures such as treatment with a chemical mutagen, for example, nitrosoguanidine, ultraviolet radiation and the like, or gene recombination and the like. In particular, a microorganism of Pseudogluconobacter saccharoketogenes is preferred. Examples thereof include the following microorganisms disclosed EP-A 221,707.

Pseudogluconobacter saccharoketogenes K591s strain: FERM BP-1130, IFO 14464
Pseudogluconobacter saccharoketogenes 12-5 strain: FERM BP-1129, IFO 14465
Pseudogluconobacter saccharoketogenes TH14-86 strain: FERM BP-1128, IFO 14466
Pseudogluconobacter saccharoketogenes 12-15 strain: FERM BP-1132, IFO 14482
Pseudogluconobacter saccharoketogenes 12-4 strain: FERM BP-1131, IFO 14483
Pseudogluconobacter saccharoketogenes 22-3 strain: FERM BP-1133, IFO 14484

In the present invention, microbial cells of a microorganism of Pseudogluconobacter per se can be used. Alternatively, their processed materials can also be used. Examples of the processed materials include a culture broth of the microorganism. In addition, enzymes produced by the microorganism can be also used. In the case of a microorganism of Pseudogluconobacter, normally, the enzyme is accumulated intracellularly. Conveniently, in the present invention, microbial cells per se are used and they are contacted and reacted with a saccharide starting material to form the corresponding carboxylic acid or a salt thereof. In particular, it is preferred to use resting cells. Such cells and culture broth can be prepared in accordance with a per se known method, for example, the method described in U.S. Patent 4,877,735 (JP-A 64-85088) or a method analogous thereto.

That is, a seed culture is prepared from a slant culture and main cultivation is carried out to obtain a culture broth. If necessary, the culture broth is centrifuged, followed by collecting the precipitate and rinsing the precipitate several times with a saline solution. The resulting precipitate can be used for the microbial reaction. The microorganism can be cultivated under aerobic conditions in a liquid medium containing assimilable nutrients, that is, carbon sources such as carbohydrates (e.g., glucose, sucrose, starch, etc.) or organic materials (e.g., peptone, yeast extract, etc.); nitrogen sources such as inorganic and organic nitrogen containing compounds (e.g., ammonium salts, urea, corn steep liquor, peptone, etc.); inorganic salts such as salts of potassium, sodium, calcium, magnesium, iron, manganese, cobalt, copper, phosphate, thiosulfate, etc. and. as trace nutrients, vitamins and coenzymes such as CoA, pantothenic acid, biotin, thiamin, riboflavin, FMN (flavin mononucleoside), etc., amino acids such as L-cysteine, L-glutamic acid, etc. or natural substances containing them. The culture broth per se thus obtained can be used in the present invention. The cultivation is carried out at pH 4 to 9, preferably, pH 6 to 8.

Although the cultivation time varies depending upon a particular microorganism used, a particular composition of culture medium and the like, preferably, cultivation is carried out for 10 to 100 hours. Suitably, the cultivation is carried out by incubation at 10 to 40°C, preferably, 25 to 35°C. Upon cultivation, when (a) rare earth element(s) is (are) added to a culture medium, the desired product can be produced more efficiently. Examples of rare earth elements to be added to the culture medium include scandium (Sc), yttrium (Y), lanthanum (La), cerium (Ce), praseodymiumu (Pr), neodymium (Nd), samarium (Sm), europium (Eu), gadolinium (Gd), terbium (Tb), dysprosium (Dy), holmium (Ho), erbium (Er), thulium (Tm), ytterbium (Yb), lutetium (Lu) and

the like. These rare earth elements can be added in the form of powdered metals or metallic pieces, or they can be used as compounds containing them such as chlorides, carbonates, sulfates, nitrates, oxides and oxalates. They can be used alone or in combination thereof, for example, cerium carbonate and lanthanum chloride can be used, simultaneously. In addition, a crude material obtained during the separation and purification of these elements can also be used. The amount of rare earth element or elements can be selected from a range in which the growth of the microorganism to be used is not inhibited and, normally, the range of 0.000001 to 0.1% (w/v), preferably, 0.0001 to 0.05% (w/v) is effective. The element or elements can be added to the culture medium beforehand. Alternatively, they can be continuously or intermittently added to the culture medium during incubation.

When the dextran carboxylic acid is produced and accumulated in the culture medium, as described in EP-A 221,707, a more increased amount of the desired product can be accumulated by cultivating the microorganism of Pseudogluconobacter in the presence of one or more other species of bacteria rather than the microorganism of Pseudogluconobacter alone. Such a mixed culture, i.e., cultivation of the microorganism of Pseudogluconobacter with one or more species of other bacteria concomitant, can be appropriately carried out in accordance with the disclosure of EP-A 221,707. As such concomitant bacteria, among others, preferred are bacteria belonging to Bacillus megaterium.

For producing the dextran carboxylic acid according to the present invention, a saccharide to be subjected to the reaction is dissolved or suspended in water or a water miscible solvent, for example, methanol, acetone, polyethylene glycol and the like and the solution or suspension can be contacted with the microorganism. The amount of the solvent to be used is not specifically limited in so far as the reaction is not delayed and, normally, the substrate concentration range of 0.1 to 20% (w/v), preferably, 1 to 5% (w/v) is effective. The microbial oxidation reaction of the present invention is suitably carried out at 10 to 40°C, preferably, 25 to 35°C. Preferably, the reaction is carried out under aerobic conditions and, for example, is carried out by aeration at a rate of 0.1 to 5 liters/minute, if necessary, with stirring at 50 to 2,000 r.p.m. Although the reaction time is varied depending upon the nature of the primary hydroxy group of the saccharide used, the reaction is carried out for 5 minutes to 3 days, normally, 1 to 24 hours. The reaction pH is preferably adjusted. Normally, the reaction is carried out at pH 4 to 9, preferably, at pH 6 to 8. Any base can be used for adjusting the reaction pH in so far as the reaction is not inhibited. For example, inorganic bases such as sodium hydroxide, potassium hydroxide, calcium carbonate, magnesium hydroxide, ferrous hydroxide and the like and organic bases such as sodium morpholinoethane sulfonate, calcium morpholinoethane sulfonate and the like can be used.

Optionally, the reaction can also be selectively controlled without using a neutralizing agent such as the above alkaline metal salt for adjusting pH by addition of an anion exchange resin. In particular, when the reaction is selectively carried out to obtain one equivalent oxidized product, this addition of an anion exchange resin is preferred. Any anion exchange resin can be used in so far as it can adsorb the carboxylic acid produced. In particular, styrene and acrylic anion exchange resins are preferred. Examples thereof include Amberlite (tradename, Organo Corp.) IRA-400, IRA-401, IRA-402, IRA-410, IRA-900, IRA-910, IRA-35, IRA-68, IRA-94S and the like, and Diaion (tradename, Mitsubisi Kasei Corp.) SA-10A, SA-20A, PA-306, PA-308, PA-406, WA-10, WA-11, WA-20, WA-30 and the like. When the saccharide added as the substrate, i.e., the dextran having hydroxymethyl group and/or hemiacetal hydroxyl moiety has been disappeared in a reaction mixture, stirring is stopped, the anion exchange resin is separated from the reaction mixture and the anion exchange resin is eluted with a suitable solvent to obtain the desired oxidized product. Examples of the eluent include an aqueous solution such as saline solution or an aqueous solution of an alkaline metal salt; or an acidic aqueous solution of hydrochloric acid, sulfuric acid, phosphoric acid, citric acid or the like.

The dextran carboxylic acid thus eluted and accumulated can be collected and purified according to a known method or its modification.

In addition, in order to remove inorganic ions from the resulting dextran carboxylic acid, it can be passed through an ionic exchange resin or subjected to desaltation treatment by dialysis, electrodialysis or ultrafiltration.

Typical examples of the dextran carboxylic acid thus obtained are represented by the formula:

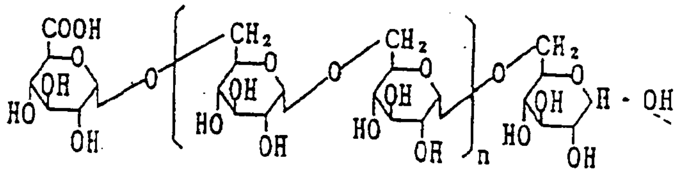

(II)

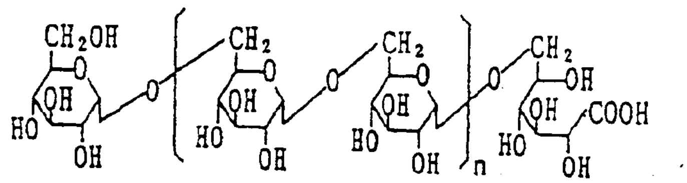

(III)

or

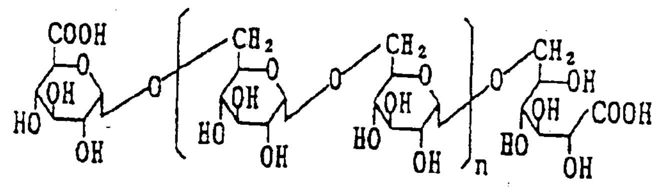

(IV)

wherein n is 1 to 50, preferably, 1 to 15.

In practice, the dextran to be used in the present invention is a mixture of polymers having different molecular weights and, preferably, the dextran is that represented by the formula:

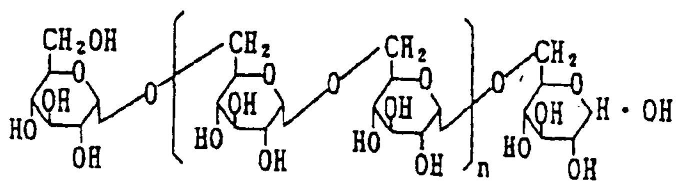

(I)

wherein n is as defined above.

The substrate to be subjected to the microbial oxidization reaction in the present invention may be or may contain a dextran derivative such as dextranyl gluconic acid or dextranyl glucuronic acid as shown by the above formula (II) or (III) or a mixture thereof and they are oxidized by the above microbial process to obtain the different oxidized product such as that shown by the above formula (IV).

The dextran carboxylic acid thus obtained can readily form a complex with an iron salt and thereby a non-toxic, stable and injectable liquid complex containing 5 to 30% or more of iron can be obtained. Examples of the iron salt include ferrous hydroxide, ferric hydroxide, iron oxide and the like. Normally, ferric hydroxide is preferred.

The complex of the dextran carboxylic acid and the iron salt can be produced according to a known method or a its modification. For example, the dextran carboxylic acid can be reacted with a sol of ferric hydroxide to obtain the complex. Preferably, the dextran carboxylic acid and the sol are subjected to heat treatment at pH 8 to 10 with, for example, an autoclave at 100 to 120°C for 30 minutes to obtain a colloidal solution or suspension.

According to this method, the dextran carboxylic acid forms a complex with the iron salt by processes of salt formation, chelating, hydration and the like. As described hereinafter, the content of iron element in the colloidal complex suspending in an aqueous phase is about 50 to 300 mg/ml of the solution or suspension and, optionally, the iron content can be increased by evaporation, concentration or the like.

The complex having high iron content thus obtained per se is very stable for a long period of time and it maintains its colloidal state, especially in an aqueous phase.

In particular, it has been found that the complex of the dextran carboxylic acid having a lower molecular weight such as that represented by the above formula (II), (III) or (IV) wherein n is 1 to 15 with an iron salt is preferred because of its desired physical properties. Among them, the complex of low molecular weight dextranyl gluconic acid and iron salt is particularly preferred and such complex can provide a stable preparation having a low viscosity and a high iron content. In fact, the iron content of a conventional dextran iron preparation is at most about 200 mg/ml, whereas it is possible to produce a preparation having a high iron content as high as about 300 mg/ml by using the complex of low molecular weight dextranyl gluconic acid and iron salt of the present invention. The high iron content is advantageous because it can decrease a dose, which improves injection work and shortens administration time. In addition, the high iron content can provide a preparation which can readily deal with a large amount of suckling pigs resulting from improvement in rearing.

According to the present invention, the complex of dextran carboxylic acid and iron salt can be diluted with injectable distilled water, physiologically saline solution or the like to produce an injectable preparation for parenteral administration to animals. Alternatively, according to a known method for preparing a veterinary composition, the complex of the present invention can be combined with a veterinary acceptable carrier, diluent or excipient to prepare a veterinary composition for oral administration such as bolus, tablets, powder, granules, capsules, emulsion, solution, premixes, syrup and the like. The composition can be used directly or, optionally after further dispersing in a carrier, it is mixed with feeds, water and the like to administer to animals.

Thus, the antianemic veterinary composition of the present invention can be produced by, for example, optionally diluting the above complex of dextran carboxylic acid and iron salt with a solid or liquid carrier, or stabilizing by coating to obtain a preparation in the form of, for example, powder, granules, tablets, bolus, solution, emulsion, paste, capsules, premixes, injectable preparations and the like. Or, the composition can be produced by optionally diluting with a carrier and adding to feeds, water and the like. Examples of a solid carrier include lactose, sucrose, starch, wheat flour, corn flour, bran, soybean oil meal, defatted rice bran, rapeseed oil meal, okara (soybean lees from "tofu (soybean curd)" production), cellulose, yeast cells, fish meal, peanut oil meal, powdered shell, calcium carbonate and the like. Examples of a liquid carrier include water, physiological saline solution, physiologically harmless organic solvents and the like. In addition, one or more appropriate auxiliaries such as emulsifiers, dispersing agents, suspending agents, wetting agents, thickening agents, gelling agents, and solubilizers can be added in a suitable amount. The composition can be in the form of a premix. Furthermore, preservatives, germicides, fungicides, anthelmintics, antioxidants, pigments, flavors, antimicrobial agents, antibiotics, enzyme preparations, probiotics (e.g., lactobacillus preparations), antipyretics, analgesics, antiphlogistics, antiinflammatory drugs and the like can also be formulated and other agents for preventing or treating anemia can be used together. Moreover, various vitamins, minerals, amino acids and the like can be formulated.

In the composition of the present invention, the amount of the complex is normally 0.1 to 100% by weight, preferably, 1 to 10% by weight based on the total weight of the composition.

The complex of the present invention is preferably administered by injection such as intramuscular injection, subcutaneous injection, intraperitoneal injection or the like. Intramuscular injection is particularly convenient. For such injection, normally, it is preferred to use the complex of the dextran carboxylic acid and the iron salt of the present invention by diluting it with injectable water, physiological saline solution or the like.

The antianemic veterinary composition of the present invention is administered to various domestic or pet animals such as pigs, cows, horse, dogs, cats and the like for preventing or treating anemia caused by not only iron deficiency but also various diseases. The dosage is varied depending upon a particular subject, disease conditions, administration route and the like. However, normally, one injection dose is selected from the range of from 5 to 500 mg of iron per one animal, preferably, 100 to 300 mg of iron per one animal. If necessary, the number of administration can be increased according to particular disease conditions. And, in the case of solid preparations such as tablets and the like, a dose corresponding to the above dose for injection can be adopted and they can be administered as they are or by mixing with feeds and the like.

Especially, the antianemic veterinary composition of the present invention is useful for preventing or treating anemia of piglets by intramuscular injection. For example, when the composition of the present invention can be intramuscularly injected at the rump, uniform dispersion and good absorption of the effective component to the tissues can be realized. In addition, the antianemic composition of the present invention does not cause any significant abnormality such as side effects and the like and the toxicity thereof is very low. For example, as described hereinafter, when the composition of the present invention has been administered to piglets, no side effect and other abnormality has been observed for 4 weeks after injection.

As seen from the results of hematological test in the experiment for administering the antianemic veterinary composition of the present invention to suckling pigs hereinafter, significant improvements have been observed with respect to many of various parameters such as, particularly, amount of hemoglobin (HGB), hematocrit value (HCT), mean red blood cell volume (MCV), body weight gain and the like in comparison with the control group.

The following Reference Examples and Examples further illustrate the present invention in detail but are not to be construed to limit the scope thereof.

Reference Example 1

Preparation of culture broth of Pseudogluconobacter saccharoketogenes TH14-86 strain

One loopful of a slant culture of Pseudogluconobacter saccharoketogenes TH14-86 (FERM BP-1128)

strain was inoculated into the each culture medium (20 ml) as shown in Table 1 and the culture medium in a 200 ml smooth flask was incubated with stirring on a rotary shaker at 30°C for 1 day. Then, a 1 ml portion from the each culture medium (20 ml) was incubated with shaking at 30°C for 2 days to obtain a seed culture. On the other hand, one loopful of a slant culture of Bacillus megaterium (IFO 12108) was inoculated into the culture medium as shown in Table 2 and the culture medium in a 200 ml smooth flask was incubated with shaking at 30°C for 2 days.

Table 1

| Culture medium for Pseudogluconobacter saccharoketogenes TH14-86 | |
|---|---|
| Components | % (w/v) |
| Lactose | 1 |
| Yeast extract (Prodivel) | 1 |
| $(NH_4)_2SO_4$ | 0.3 |
| Corn steep liquor | 3 |
| $CaCO_3$ (Super) | 2 |

pH before addition of $CaCO_3$ = 7.0

Table 2

| Culture medium for Bacillus megaterium | |
|---|---|
| Components | % (w/v) |
| Sucrose | 4 |
| Proflo [N source, Tradas protein (T & P)] | 4 |
| $K_2HPO_4$ | 0.65 |
| $KH_2PO_4$ | 0.55 |
| NaCl | 0.05 |
| $(NH_4)_2SO_4$ | 0.05 |
| $MgSO_4 \cdot 7H_2O$ | 0.005 |
| Calcium pantotenate | 0.025 |

pH before sterilization = 7.0

Then, the following main cultivation was carried out.

The above seed culture of Pseudogluconobacter saccharoketogenes TH14-86 (100 ml) and the seed culture of Bacillus megaterium (1.5 ml) were inoculated into the production culture medium (900 ml) as shown in Table 3 and incubated with shaking at 30 °C for about 20 hours to obtain a culture broth of Pseudogluconobacter saccharoketogenes TH14-86.

Table 3

| Main culture medium | |
|---|---|
| Components | % (w/v) |
| Sucrose | 0.05 |
| | independently sterilized |
| Corn steep liquor | 2 |
| | independently sterilized |
| $(NH_4)_2SO_4$ | 0.3 |
| | independently sterilized |
| $FeSO_4 \cdot 7H_20$ | 0.1 |
| | independently sterilized |
| Vitamin $B_2$ | 1 mg/liter |
| pH before sterilization = 7.0 | |
| Sorbose | 10 |
| | independently sterilized |
| $LaCl_3$ | 0.01 |
| | independently sterilized |
| $CaCO_3$ (Super) | 4 |
| | independently sterilized |

The culture broth of Pseudogluconobacter saccharoketogenes TH14-86 thus obtained (1.5 liters) and Dextran-4 (molecular weight: 4,000 - 6,000; manufactured by Extrasynthese Corp., France) (30 g) were placed in a 5 liter jar fermentor (manufactured by Biott Co.) and sterilized water (1. 7 liters) was added thereto to obtain a reaction mixture. This was aerated at 1.6 liters/minute with stirring at 600 r.p.m. and aqueous 0.5N NaOH solution was automatically added dropwise with progress of the reaction to adjust pH at 6.3. The reaction was stopped after 3 hours and the reaction mixture (12.0 liters) was centrifuged with a cooling centrifuge at 8,000 r.p.m. to remove microbial cells to obtain a supernatant (1.98 liters). The supernatant was filtered through a cellulose acetate membrane filter (ϕ 0.2- μm) to further remove microbial cells. The filtrate was passed through a column of Amberlite IRA-68 (OH type) (50 ml) and washed with a small amount of water (100 ml) to obtain a solution (2 liters). This solution was passed through a HP-20 column (900 ml) and the column was eluted with water (2 liters). A solution initially passed through the column (700 ml) was discarded and the subsequent eluent (3.3 ml) was collected. Conc. hydrochloric acid (13.8 ml) was added to the eluent to make it acidic and the solution was filtered through a cellulose acetate membrane filter (ϕ 0.2 μm). The filtrate was placed on a column of Cephabeads SP205 (tradename, Mitsubishi Kasei Corp.) (1,000 ml), washed with 0.05M hydrochloric acid 700 ml) and water (2 liters), and then eluted with 20% aqueous methanol (2 liters) to obtain the desired fraction. The fraction was concentrated under reduced pressure to obtain white powder of dextranyl glucuronic acid (14 g). The HPLC [conditions: Asahi Pack GS320 (ϕ 7.6 mm x 50 mm, manufactured by Ashai Chemical Industry Co., Ltd.), mobile phase; water 1 ml/min., detection; RI (Waters 410) and 200 nm (Toso UV-8020), injection; aqueous 0.5% sample solution 20 μl] of this product showed a single peak at Rt of 8.48. Rt of Dextran-4 used as the starting material was 10.83.

In order to confirm the structure of this product, enzymatic digestion was carried out. A solution of glucoamylase (manufactured by Wako Pure Chemical Industries, Ltd.) (4 mg/ml, 100 μl) was added to a 1.5% aqueous solution of this product (100 μl) and the mixture was incubated at 30°C for one day and night. The solution thus treated with the enzyme was studied by HPLC. As a control, Dextran-4 was also treated with the enzyme according to the same manner. As a result, it has been found that this product was not digested by the enzyme,

whereas Dextran-4 was digested by the enzyme and disappeared to form glucose. In view of this result, this product was confirmed to be dextranyl glucuronic acid of the above formula (II) wherein n being on the average 8.

Reference Example 2

Pseudogluconobacter saccharoketogenes K591s (FERM BP-1130) strain was cultivated in a peptone yeast (PY) culture medium comprising bactopeptone 1% and yeast extract 1% with shaking at 230 r.p.m. at 30°C for 3 days. pH was adjusted to 7.5. Then, the main cultivation was carried out in a PY culture containing 0.1% potassium chloride with shaking at 230 r.p.m. at 30°C for 48 hours. Then, the resulting culture broth was centrifuged at 10,000 r.p.m. for 10 minutes and microbial cells were collected and rinsed with water (500 ml) to obtain the microbial cells (7.59 g/ 1 liter of culture broth). Then, Dextran-4 (molecular weight: 4,000 - 6,000, Extrasynthese Corp., France) (30 g) was dissolved in water (1 liter) and the wet cells (56 g) suspended in water (1 liter) was added thereto. The mixture was reacted with stirring at 800 r.p.m. and aerating at 60 ml/min. at 32 °C for 6.5 hours, while adjusting pH at 6.3 with aqueous 0.1% NaOH solution. The reaction mixture was centrifuged to obtain a supernatant (2 liters). The supernatant was filtered through a membrane filter to remove microbial cells and the filtrate was placed on a HP-20 column (900 ml) and eluted with water (2,000 ml). The eluent was made acidic by addition of conc. hydrochloric acid so that the final concentration was 0.05M. Then, the eluent was filtered with a membrane filter and the filtrate was placed on a SP-205 (100 ml) column, washed with 0.05M HCl (1,000 ml) and then water (2,000 ml), and eluted with 20% aqueous methanol (2,000 ml). The methanol fraction was collected, concentrated and lyophilized to obtain white powder of dextranyl gluconic acid (14 g). The HPLC [conditions: Asahi Pack GS320 (ϕ 7.6 mm x 50 mm, manufactured by Ashai Chemical Industry Co., Ltd.), mobile phase; water 1 ml/min., detection; RI (Waters 410) and 200 nm (Toso UV-8020), injection; aqueous 0.5% sample solution 20 μl] of this product showed a single peak at Rt of 8.70. Rt of Dextran-4 used as the starting material was 10.83. When this product was subjected to glucoamylase digestion according to the same manner as described above, it was readily digested and the formation of glucose and glucosyl gluconic acid were detected. Therefore, this product was confirmed to be dextranyl gluconic acid of the above formula (III) wherein n being on the average 8.

Reference Example 3

According to the same manner as described in Reference Example 2, Pseudogluconobacter saccharoketogenes was cultivated and its wet cells (50 g) was added to a solution of dextranyl glucuronic acid prepared in Reference Example 1 (30 g) in water (1 liter) and the mixture was reacted with stirring at 800 r.p.m. and aerating at 60 ml/min. at 32°C for 21 hours, while adjusting pH at 6.3 with aqueous 0.1% NaOH solution. The reaction mixture was centrifuged to obtain a supernatant (2 liters). The supernatant was filtered through a membrane filter to remove microbial cells, placed on a HP-20 (900 ml) column and eluted with water (1.5 liters). The desired fraction was collected, concentrated and lyophilized to obtain white powder of sodium glucuronyl dextranyl gluconate (12 g).

Reference Example 4

An aqueous 50% solution of ferric chloride hexahydrate ($FeCl_3 \cdot 6H_2O$) (50 ml) was warmed at 30°C and an aqueous 24% solution of $Na_2CO_3$ (50 ml) was added dropwise at the rate of 0.4 ml/min. with vigorous stirring. Then, a 10% solution of dextranyl glucuronic acid obtained in Reference Example 1 (50 ml) was added dropwise at the rate of 3 ml/min., while aqueous 16% solution of $Na_2CO_3$ was added dropwise at the rate of 0.4 ml/min. to adjust pH at 4.3. Ethanol (200 ml) was added thereto and vigorously stirred to obtain a slurry. The slurry was centrifuged (5,000 r.p.m.) and the precipitate was collected and dissolved in water (40 ml). The solution was vigorously stirred together with ethanol (60 ml) and the mixture was centrifuged to remove soluble materials. The precipitate was dispersed in water (20 ml) and ethanol was removed with an evaporator under reduced pressure. An aqueous 10% NaOH solution was added at the rate of 0.2 ml/min. with vigorous stirring to adjust pH 5 to 6 and the mixture was heated at 100°C for 20 minutes. Then, phenol was added so that its concentration became 4 mg/ml to obtain the desired complex of dextranyl glucuronic acid and ferric hydroxide sol solution (22 ml).

Reference Example 5

Ferric chloride hexahydrate (100 g) was dissolved in water (100 ml) and the solution was sonicated with

Branson G-220 for 1 hour to dissolve the ferric chloride sufficiently. Water was added thereto to adjust the volume to 200 ml and the solution was transferred to a 500 ml beaker. An aqueous 24% $Na_2CO_3$ solution (200 ml) was added at the rate of 0.8/min. at 30°C with vigorous stirring to obtain a pale yellowish brown ferric hydroxide sol.

The ferric hydroxide sol (100 ml) thus prepared was collected and transferred to a 300 ml beaker. An aqueous 10% dextranyl gluconic acid solution (50 ml) was slowly added thereto with vigorous stirring at 30°C. Then, an aqueous 16% $Na_2CO_3$ solution was very slowly added thereto at the rate of 0.1 to 0.2 ml/min. to adjust pH at 4.3. Ethanol (200 ml) was added with stirring and the precipitate thus formed was centrifuged to separate a supernatant. The precipitate was suspended in water (80 ml) and ethanol (120 ml) was added to reprecipitate the product. The precipitate was collected by centrifugation. This operation was repeated once and the resulting precipitate was suspended in water (20 ml) and vigorously stirred. An aqueous 10% NaOH solution was added at the rate of 0.1 to 0.2 ml/min. until pH became 6.0. The solution was autoclaved at 120°C for 10 minutes and phenol as a preservative (the final concentration of 1%) was added and the mixture was concentrated with an evaporator to obtain the desired complex of dextranyl gluconic acid and ferric hydroxide sol. This product forms stable ferric hydroxide sol. Properties thereof are as follows. Total iron salt concentration: 206 mg/ml, Viscosity: 32 cP, Electric conductance: 47 mS/cm.

Example 1

Samples tested

The complex of dextranyl glucuronic acid and ferric hydroxide sol obtained in Reference Example 4 (containing 129 mg Fe/ml, hereinafter referred to as glucuronic acid/iron) and the complex of dextranyl gluconic acid and ferric hydroxide sol obtained in Reference Example 5 (containing 206 mg Fe/ml, hereinafter referred to as gluconic acid/iron) were used as the antianemic veterinary agents.

Animals tested

Landrace suckling piglets were used.

Test method

Nine litters of a pig of 6 days old were separated into 3 groups (n = 3) and 200 mg Fe/pig of the sample was intra-muscularly injected at the left rump. The pigs were weighed and blooded at the initiation of the test and once every week thereafter. Parameters, i.e., amount of hemoglobin (HGB), hematocrit value (HCT), mean red blood cell volume (MCV) and body weight were determined and compared with an untreated control group. The results are shown in Table 4.

Table 4

| Parameter | Sample | Weeks 0 | 1 | 2 | 3 | 4 |
|---|---|---|---|---|---|---|
| HGB (g/dl) | Control | 7.8 | 5.1 | 5.2 | 6.8 | 6.0 |
| | Glucuronic acid/iron | 7.0 | 5.9 | 6.7 | 8.2 | 7.7 |
| | Gluconic acid/iron | 7.4 | 8.2* | 9.2* | 11.2* | 9.7 |
| HCT (%) | Control | 28.3 | 19.5 | 19.6 | 27.7 | 24.6 |
| | Glucuronic acid/iron | 24.9 | 22.7* | 24.3 | 30.1 | 29.0 |
| | Gluconic acid/iron | 26.9 | 31.2* | 31.6* | 39.5* | 33.7 |
| MCV (fl) | Control | 72.0 | 59.1 | 47.0 | 47.4 | 44.5 |
| | Glucuronic acid/iron | 73.2 | 66.0* | 55.0* | 49.3 | 45.6 |
| | Gluconic acid/iron | 73.9 | 82.2* | 68.0* | 62.5* | 57.9* |
| Body weight (kg) | Control | 2.25 | 4.08 | 5.55 | 6.88 | 6.98 |
| | Glucuronic acid/iron | 2.30 | 4.20 | 6.02 | 7.37 | 7.30 |
| | Gluconic acid/iron | 2.27 | 4.13 | 5.90 | 7.53 | 7.67 |

*: $p<0.05$ significant

As seen from Table 4, in comparison with the control group, each anemic parameter of blood is significantly improved and inhibition of progress of anemia by administration of the complex of the present invention is clearly shown.

In addition, the serum iron level and total iron-binding capacity 3 weeks after administration were determined and unsaturated iron-binding capacity (= total iron-binding capacity - serum iron) and iron saturation degree (= serum iron/total iron-binding capacity x 100) were calculated and compared with the control group. The results are shown in Table 5.

Table 5

| Sample | Serum iron (μg/dl) | Total iron-binding capacity (μg/dl) | Unsaturated iron-binding capacity (μg/dl) | Saturation degree (%) |
|---|---|---|---|---|
| Control | 60.5 | 755.8 | 695.3 | 8.1 |
| Glucuronic acid/iron | 101.5 | 646.4 | 544.8 | 15.7 |
| Gluconic acid/iron | 152.8 | 588.1 | 435.3 | 27.8 |

As seen from Table 5, in comparison with the control group, when administered the complex of the present invention, the serum iron level is remarkably high and increase in total iron-binding capacity is inhibited. Thereby, the saturation degree is clearly improved. This clearly shows that the antianemic complex of the present invention is efficiently absorbed from the injection tissue and utilized. Thus, it is clear that the composition of the present invention is effective for preventing and treating anemia of animals.

Reference Example 6

According to the same manner as described in Reference Example 2, rinsed microbial cells of Pseudogluconobacter saccharoketogenes K591s strain were obtained. Dextran-4 (molecular weight: 4,000 - 6,000, manufactured by Extrasynthase Corp., France) (30 g) was dissolved in water (1 liter) and the solution was added to the wet microbial cells (56 g) suspended in water (1 liter). The mixture was reacted with stirring at 800 r.p.m. and aerating at the rate of 60 ml/min. at 32°C for 5 hours, while adjusting pH to 6.3 with an aqueous 0.1% NaOH solution. The reaction mixture was analyzed by HPLC under the same conditions as those in Reference Example 2 and it was found that the product was composed of 62% of dextranyl gluconic acid having Rt of 8.70 and 38% of un-oxidized dextran having Rt of 10.90. The reaction mixture was centrifuged and the supernatant (2 liters) was collected. The supernatant was filtered through a membrane filter to remove the microbial cells, placed on an IR-120B ($H^+$) column (100 ml) and eluted with water (200 ml). The eluent was collected, concentrated under reduced pressure and lyophilized to obtain white powder (27 g). According to HPLC, it was confirmed that this product was a mixture of dextranyl gluconic acid and dextran in the ratio of 6 : 4.

Then, according to the same manner as described in Reference Example 5, a ferric hydroxide sol was prepared and a 100 ml portion thereof was transferred into a dialysis tube, SPECTRA/POR 2 (cut off molecular weight: 12,000 - 14,000, manufactured by SPECTRUM MEDICAL INDUSTRIES, Inc., U.S.A.) and dialyzed for overnight. To the solution of the iron hydroxide sol obtained after dialysis was slowly added a solution of the above white powder (the 6 : 4 mixture of dextranyl gluconic acid and dextran) (5 g) in water (50 ml) with vigorous stirring at 30°C. Then, an aqueous 16% $Na_2CO_3$ solution was very slowly added to adjust pH to 4.3. After stirring at 30°C for 1 hour, the mixture was autoclaved at 100°C for 30 minutes to obtain a solution. The pH of the solution was adjusted to 6.41 with 1N NaOH solution, concentrated with an evaporator. As a preservative, phenol (the final concentration of 0.5%) was added to obtain the desired complex of dextranyl gluconic acid and iron hydroxide sol. This product formed a stable hydroxide sol. The properties thereof were as follows. Total iron concentration: 197 mg/ml, Viscosity: 62 cP, Electric conductance: 50 mS/cm.

Example 2

Antianemic activity of the complex of Reference Example 6 was tested by using Landrace pigs and as a control agent Anemex (tradename of a commercially available antianemic preparation containing dextran iron of 200 mg Fe/ml, manufactured by Fujita Pharmaceutical Co., Ltd.).

The complex or the control agent (200 mg Fe/pig) was intramuscularly injected at the rump of the 2 pigs of 3 days old respectively. After 2 weeks, the amount of hemoglobin (HGB), hematocrit value (HCT), mean red blood cell volume (MCV) and body weight gain were determined. The results are shown in Table 6 as the relative index by taking the corresponding parameter of the control agent as 100.

Table 6

| Parameter | Control agent | Present invention |
|---|---|---|
| HGB | 100 | 109 |
| HCT | 100 | 110 |
| MCV | 100 | 106 |
| Weight gain | 100 | 115 |

As seen from Table 6, in comparison with the control agent, when the complex of the present invention is used, anemic parameters were improved.

Reference Example 7

One loopful of a slant culture of Pseudogluconobacter saccharoketogenes K591s strain was inoculated into a test tube (16 mm x 160 mm) containing the complete culture medium (5 ml) as shown in Table 7 and incubated with shaking at 30°C for 2 days.

Table 7

| Complete culture medium | |
|---|---|
| Components | g/liter |
| D-sorbitol | 25 |
| Peptone | 10 |
| Yeast extract | 10 |

pH = 6.5 (In the case of a solid medium, 20 g/liter of agar is added.)

This culture broth (1 ml) was transferred into another test tube containing the same culture medium (5 ml) and cultivated with shaking for 5 hours. The culture broth thus obtained (5 ml) was aseptically centrifuged (6,500 r.p.m.) at 5°C for 15 minutes to collect the microbial cells. Then, the microbial cells were suspended in 0.05M Tris-maleate buffer (10 ml, pH 6.5) and again centrifuged (6,500 r.p.m.). This operation was repeated twice. The resulting washed microbial cells was suspended in the same buffer (5 ml) containing N-methyl-N'-nitro-N-nitrosoguanidine (nitrosoguanidine) (0.5 mg/ml) and incubated with shaking at 30°C for 1 hour. The mixture was centrifuged (6,500 r.p.m.) at 5°C for 15 minutes to collect the microbial cells. According to the same manner as the above, the microbial cells were washed twice with a Tris-maleate buffer (10 ml) to obtain the microbial cells treated with nitrosoguanidine. The cells were appropriately diluted with 0.85% saline solution, seeded on a plate (9 cm in diameter) containing the complete culture medium (solid, 15 ml) and cultivated at 30°C for 5 days to form colonies. The colony on the complete culture medium was replicated on a plate of the minimum culture medium (solid) as shown in Table 8 and incubated at 30 °C for 3 days.

Table 8

| Minimum culture medium | |
|---|---|
| Components | g/liter |
| Sucrose | 5 |
| $K_2HPO_4$ | 3 |
| $KH_2PO_4$ | 1 |
| $(NH_4)_2SO_4$ | 1 |
| NaCl | 1 |
| $MgSO_4 \cdot 7H_2O$ | 0.1 |
| $MnCl_2 \cdot nH_2O$ | 0.002 |
| Sodium L-glutamate | 0.1 |
| L-cysteine | 0.1 |
| CoA | 0.002 |
| FMN | 0.002 |
| Thiamin | 0.002 |
| Biotin | 0.001 |
| Agar | 20 |

pH = 7.0

Then, the resulting culture broth was appropriately diluted and seeded on a plate of the selection culture medium (solid) as shown in Table 9 so that about 50 colonies were formed on a plate.

Table 9

| Selection culture medium | |
|---|---|
| Components | g/liter |
| L-sorbose | 5 |
| Peptone | 5 |
| Yeast extract | 2 |
| $CeCl_3 \cdot 7H_2O$ | 0.01 |
| $CaCO_3$ | 2 |
| Agar | 20 |

pH = 7.0

After incubation at 30°C for 12 days, the colony about which any calcium carbonate dissolved area was recognized was selected.

According to the same culture medium and conditions as described in Reference Example 1, a culture broth of the above treated microorganism was prepared and the rinsed microbial cells thereof were obtained. Then, dextran (molecular weight: 1,500; manufactured by BioChemika AG, Switzerland) (30 g) were dissolved in water (1 liter) and the wet microbial cells (40 g) suspended in water (1 liter) was added thereto. This was reacted with stirring at 800 r.p.m. and aerating at 60 ml/min. at 32°C for 21 hours, while adjusting the pH to 6.3 with

an aqueous 0.1% NaOH solution. When the reaction mixture was analyzed by HPLC under the same conditions as those in Reference Example 2, it was found that the mixture was composed of 96% of dextranyl gluconic acid having Rt of 9.69 and 4% of unoxidized dextran having Rt of 15.12.

Then, the reaction mixture was centrifuged and the supernatant was collected, filtered through a membrane filter to remove microbial cells and placed on a IR-120B ($H^+$) column (100 ml). The column was eluted with water (200 ml) and the eluent was collected, concentrated under reduced pressure and lyophilized to obtain white powder (29 g). The HPLC of this product showed that it was a mixture of dextranyl gluconic acid and dextran in the ratio of 90 : 10.

According to the same manner as described in Reference Example 5, iron hydroxide sol was prepared and a 100 ml portion thereof was placed in a dialysis tube, SPECTRA/POR (SPECTRUM MEDICAL INDUSTRIES Inc., U.S.A.) and dialyzed for overnight. After dialysis, to the ferric hydroxide sol solution was slowly added to the above white powder (90 : 10 mixture of dextranyl gluconic acid and dextran) (5 g) dissolved in water (50 ml) with vigorously stirring at 30°C. Then, an aqueous 16% $Na_2CO_3$ solution was slowly added to adjust pH to 4.3. The mixture was further stirred at 30°C for 1 hour and autoclaved at 100°C for 30 minutes to obtain a solution. This solution was adjusted to pH 6.40 with 1N NaOH solution and concentrated with an evaporator. As a preservative, phenol (the final concentration of 0.5%) was added thereto and the desired complex. This product was a stable ferric hydroxide sol. The properties were as follows.
Total iron salt concentration: 238 mg/ml, Viscosity: 21.9. cP,
Electric conductance: 47 mS/cm.

## Example 3

Antianemic activity of the complex of Reference Example 7 was tested by using Landrace pigs of 5 days old.

The complex was intramuscularly injected at the rump of the 2 pigs (200 mg Fe/pig). After 2 weeks, the amount of hemoglobin (HGB), hematocrit value (HCT), mean red blood cell volume (MCV) and body weight gain were determined and compared with an untreated control group. The results are shown in Table 10 as the relative index by taking the corresponding parameter of the control agent as 100.

Table 10

| Parameter | Control group | Group of Present invention |
|---|---|---|
| HGB | 100 | 309 |
| HCT | 100 | 259 |
| MCV | 100 | 143 |
| Weight gain | 100 | 133 |

As seen from Table 10, in comparison with the control group, when the complex of the present invention is used, anemic parameters were remarkably improved.

## Reference Example 8

According to the same manner as described in Reference Example 5, an ferric hydroxide sol was prepared and a 100 ml portion thereof was collected and transferred into a dialysis tube, SPECTRA/POR and dialyzed overnight. After dialysis, to the resulting ferric hydroxide sol solution was added dextranyl gluconic acid (average molecular weight: 1,500; oxidized product of dextran having average molecular weight of 1,500) (5 g) dissolved in water (59 ml) with vigorous stirring at 30°C. Then, an aqueous 16% $Na_2CO_3$ solution was very slowly added to adjust to pH 6.40 and then the mixture was autoclaved at 100°C for 30 minutes to obtain a solution. The solution was again placed in a dialysis tube, SPECTRA/POR, and dialyzed overnight. The dialyzed inner solution was concentrated with an evaporator and, as a preservative, phenol (the final concentration of 0.5%) was added to obtain the desired stable iron hydroxide sol preparation. The properties of this preparation were as follows.
Total iron salt concentration: 331 mg/ml, Viscosity: 38.0 cP, Electric conductance: 6.6 mS/cm.

As described hereinabove, the antianemic veterinary composition comprising the complex of dextran carboxylic acid and iron salt of the present invention remarkably improve anemia of animals such as pigs and the

like, and the composition is very safe because its toxicity is very low. Especially, according to the present invention, it is possible to prepare a veterinary composition stably containing a high concentration of iron and having low viscosity. Therefore, even if the antianemic veterinary composition of the present invention is in the form of an injectable preparation, it can be safely administered to suckling pigs and the like without accompanying any colored spot or pain.

## Claims

1. An antianemic composition for veterinary use which comprises a complex of a dextran carboxylic acid obtained by a microbial process with an iron salt.

2. An antianemic composition for veterinary use according to claim 1, wherein the dextran carboxylic acid is obtained by allowing a microorganism which belongs to the genus Pseudogluconobacter and is capable of oxidizing the hydroxymethyl group and/or OH-containing hemiacetal moiety of a dextran having hydroxymethyl group and/or hemiacetal hydroxyl group to carboxyl group, or its processed material to act on a dextran having such group to produce the corresponding carboxylic acid, and recovering it.

3. An antianemic composition for veterinary use according to claim 2, wherein the microorganism is Pseudogluconobacter saccharoketogenes.

4. An antianemic composition for veterinary use according to claim 2, wherein the microorganism is Pseudogluconobacter saccharoketogenes FERM BP-1128, FERM BP-1129, FERM BP-1130, FERM-BP 1131, FERM-BP 1132 or FERM-BP 1133.

5. An antianemic composition for veterinary use according to claim 1, wherein the iron salt is an iron hydroxide or an iron oxide.

6. An antianemic composition for veterinary use according to claim 5, wherein the iron salt is ferric hydroxide.

7. An antianemic composition for veterinary use according to claim 2, wherein the processed material of the microorganism is a culture broth of the microorganism.

8. An antianemic composition for veterinary use according to claim 1, wherein the dextran carboxylic acid is represented by the formula:

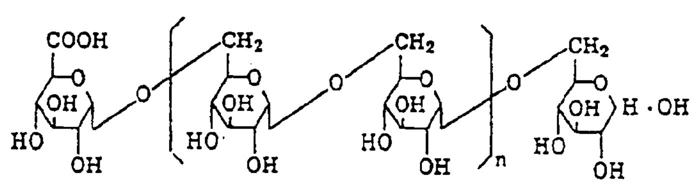

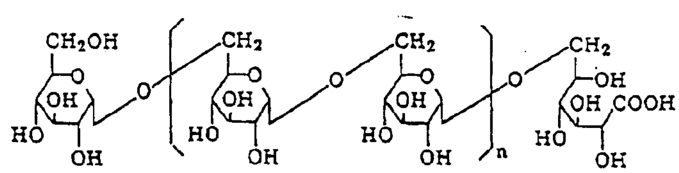

or

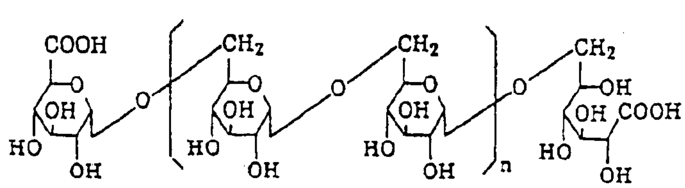

wherein n is 1 to 50.

9. An antianemic composition for veterinary use according to claim 8, wherein n is 1 to 15.

10. An antianemic composition for veterinary use according to claim 1, wherein the complex is obtained by reacting the dextran carboxylic acid with ferric hydroxide sol.

11. An antianemic composition for veterinary use according to claim 1, wherein the composition is in the form of an injectable preparation.

12. An antianemic composition for veterinary use according to claim 11, wherein the composition is in the form of an injectable preparation for intramuscular administration.

13. An antianemic composition for veterinary use according to claim 1, wherein the composition is to be used for piglets.

14. An antianemic composition for veterinary use according to claim 1, which is applied for an anemia caused by iron deficiency.

15. Use of a complex of a dextran carboxylic acid obtained by a microbial process with an iron salt as an antianemic veterinary composition for preventing or treating animal anemia.

16. A complex of a dextran carboxylic acid obtained by a microbial process with an iron salt for use as an active veterinary substance for preventing or treating animal anemia.

European Patent Office

# EUROPEAN SEARCH REPORT

Application Number
EP 94 30 5093

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,P | EP-A-0 599 646 (TAKEDA CHEMICAL INDUSTRIES LTD)<br>* page 7, line 46 - line 53 *<br>* page 7, line 58 - page 8, line 9 *<br>* page 9, line 37 - line 39 *<br>* page 26; example 21 *<br>* page 26; example 23 *<br>* page 4, line 5 - line 15 *<br>----- | 1-16 | A61K31/70<br>A61K33/26 |
| | | | TECHNICAL FIELDS SEARCHED (Int.Cl.6) |
| | | | A61K |

The present search report has been drawn up for all claims

| Place of search | Date of completion of the search | Examiner |
|---|---|---|
| THE HAGUE | 25 October 1994 | Gerli, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)